# EUROPEAN PATENT APPLICATION

(11) **EP 1 997 796 A1**
(43) Date of publication of application: **03.12.2008**
(21) Application number: 07010856.8
(22) Date of filing: 01.06.2007
(51) Int. Cl.: C07C 45/72

(54) **Aldol condensation reaction and catalyst therefore**

(71) Applicant: DSM IP Assets B.V., 6411 TE Heerlen (NL)
(72) Inventor: The designation of the inventor has not yet been filed
(74) Representative: Schwander, Kuno

(57) **Abstract**

The invention relates to a novel processes for cross aldol reactions using strongly basic, anionic, macroreticular polymeric resin with quaternary ammonium groups of the type -CH₂N⁺(CH₃)₃ as heterogeneous catalysts. This catalyst is recyclable and the process can be repeated several times.

## Description

The invention relates to a novel processes for cross aldol reactions using strongly basic, anionic, macroreticular polymeric resin with a -CH₂N⁺(CH₃)₃ active group as heterogeneous catalysts. This catalyst is recyclable and the process can be repeated several times.

Aldol reactions are important in the production of intermediates needed to synthesize many commercially important products. The addition of ketones and/ or aldehydes to obtain aldols (β-hydroxy ketones) is a well-known reaction. Dehydration of the resulting aldol to obtain an α,β-unsaturated ketone is also known. Subsequent catalytic hydrogenation of the unsaturated ketone may be carried out to obtain the corresponding saturated higher ketone. However, the starting materials, the intermediate β-hydroxy ketones as well as the α,β-unsaturated ketones are known to those skilled in the art to be quite reactive and susceptible to further consecutive, non-selective condensation, cyclization, and Michael-type addition reactions with the starting ketones and aldehydes, as well as themselves and other ketones and aldehyde by-products.

Many methods have been disclosed in the art to perform aldol condensation reactions. These methods include for example homogeneous, base catalysed aldol reactions such as disclosed in WO 2004041764 or CN 1202065. However, homogeneous catalysed aldol reactions have the disadvantage of exhibiting high salt content often resulting in an unwanted contamination of the final product. Heterogeneous catalysed reactions are also disclosed as versatile methods to catalyze aldol reactions. Heterogeneous catalysis is of fundamental importance in the chemical industry because of its "simplicity" as the catalyst can be removed via filtration.

Various anion exchange resins are described to catalyze aldol condensation reactions for example strong basic polystyrene anion exchange resins for the condensation of citral and acetone [Z. Nengfang; L., Guiyun. Lizi Jiaohuan Yu Xifu: (1991), 7(2), 142-146, L. Tianhua et al: Jiangsu Shiyou Huagong Xueyuan Xuebao (1998), 10(1), 15-18] or a fluoride ion supported on anion exchange resin [Lin, Hong-wei. Hecheng Huaxue (2004), 12(4), 402-404]. However, no detailed information about the properties of the catalyst is given.

PL 147748 discloses the preparation of ionones and methylionones by condensation of citral with acetone or methyl ethyl ketone (2-butanone) using weak basic anion exchanger catalysts. However, the catalyst has a gel structure and can not versatile be recycled.

Tianran Chanwu Yanjiu Yu Kaifa (1997), 9(3), 59-64 discloses the use of macroporous strong-base ion exchange resin for the preparation of pseudoionone. However, the needed amount of ion exchanger is large and the lifetime is short, so the process is not economical.

Thus, there is an ongoing need for a simple, economically attractive, highly selective and environmentally benign method which allows the aldol condensation products to be formed in good yields and purities and, permits the catalyst to be recycled by means of a simple industrial process and makes it possible to guarantee constantly stable yields and process conditions with repeated use of the catalyst.

As a result of extensive screening studies, it has surprisingly been found that the object of the invention is achieved by using a strongly basic, anionic, macroreticular polymeric resin having quaternary ammonium groups as active sites as catalyst for carrying out the aldol condensation reaction, especially a catalyst with a surface area of 10-100 m²/g, preferably 20-50 m²/g, in particular with a surface area 30 m²/g, and an average pore diameter of 200-500A, in particular 290 - 300 A.

Thus, the invention relates to a process for the preparation of α,β-unsaturated ketones, the process comprising the step of reacting an aldhyde of formula (1) 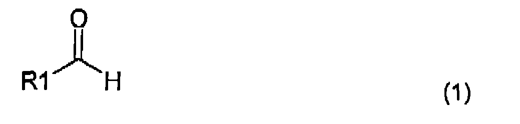 with a ketone of formula (2) 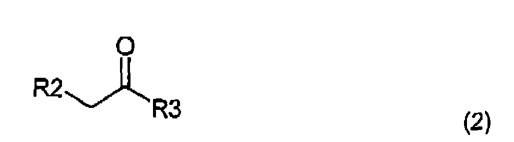 wherein
R1, R2 and R3 represent independently of each other hydrogen, a saturated or unsaturated, branched or non-branched, hydrocarbons residue with 1 to 20 C-atoms or a cycloalkyl group
with 4 to 12 C-atoms, which optionally may be substituted e.g. by one or several methoxy groups
in the presence of a strongly basic, anionic, macroreticular polymeric resin having
   a)quaternary ammonium groups of the type -CH₂N⁺(CH₃)₃ as active sites
   b) a surface area of 10 - 100 m²/g, preferably of 20 - 50 m²/g, in particular a surface area of 30 m²/g, and
   c) an average pore diameter of 200-500 A, in particular 290 - 300 A.

In the following the strongly basic, anionic, macroreticular polymeric resin according to the invention is abbreviated as "solid catalyst".

Surprisingly, it has been found that in case of citral using asymmetric methylketones a high selectivity towards the addition to the methyl group can be achieved. Thus, in a preferred embodiment the invention relates to a process for the preparation of α,β-unsaturated ketones as outlined above wherein the aldehyde is citral, R3 is methyl and R2 is selected from a saturated or unsaturated, branched or non-branched, hydrocarbons residue with 1 to 20 C-atoms or a cycloalkyl group with 4 to 12 C-atoms, which optionally may be substituted e.g. by one or several methoxy groups.

The following scheme illustrates the aldol condensation reaction according to the invention whereby the compound in bracket is passed through as intermediate.

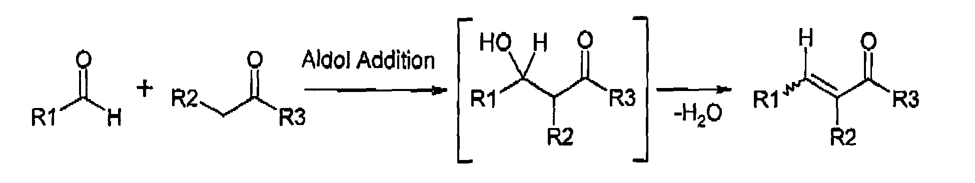

The addition of the ketone to the aldehydes results in an intermediate aldol (β-hydroxy ketones) which is dehydrated in situ to the respective α,β-unsaturated ketone. This reaction is catalysed by the solid catalyst according to the invention.

In another preferred embodiment, the invention relates to a process for the production of α,β-unsaturated ketones of formula (3) 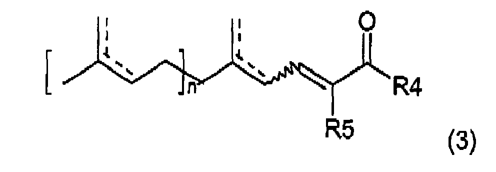 the process comprising reacting an alkyl ketone of formula (4) 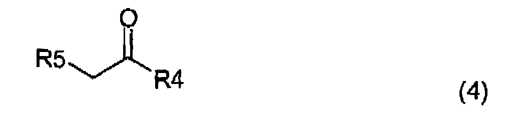 and an aldehyde of formula (5) 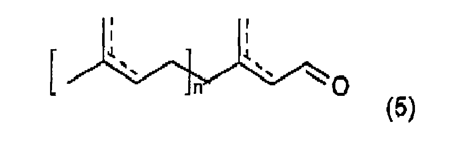 wherein
R4 represents a saturated or unsaturated, linear or branched hydrocarbons residue with 1 to 20 C-atoms, preferably a saturated or unsaturated hydrocarbons residue with 1 to 10 C-atoms, more preferably a saturated hydrocarbons with 1 to 5 C-atoms (i.e. 1,2,3,4, or 5 C-atoms), and
R5 represents hydrogen, a saturated or unsaturated, linear or branched hydrocarbons residue with 1 to 20 C-atoms or a cycloalkyl group with 4 to 12 C-atoms, which optionally may be substituted e.g. by one or several methoxy or ether groups, preferably hydrogen and
n represents a number from 0 to 4, preferably from 0 to 2 and
wherein the dotted lines indicate, independently of each other, either a saturated or a double bond and if the dotted lines represent a double bond, it can be arranged in one of the two indicated positions and
wherein the process is carried out in the presence of a strongly basic, anionic, macroreticular polymeric resin having
a)quaternary ammonium groups of the type -CH₂N⁺(CH₃)₃ as active sites and
b) a surface area of 10-100 m²/g, preferably of 20 - 50 m²/g, in particular a surface area of 30 m²/g, and
c) an average pore diameter of 200-500 A, in particular 290 - 300 A.

In all embodiments of the invention preferably the catalyst is recycled after termination of the reaction by filtration, optionally washed with an alcohol, preferably ethanol and re-used in a subsequent reaction cycle. Preferably, the process according to the invention including the recycling of the solid catalyst is repeated at least once using the recycled catalyst, even more preferably, the catalyst is re-used 2 to 10 times such as 2, 3, 4, 5, 6, 7, 8, 9 or 10 times, in particular 4 times in the process according to the invention.

In all embodiments of the invention, the solid catalyst preferably is strongly basic, anionic, macroreticular polymeric resin based on a crosslinked styrene divinylbenzene copolymer containing -CH₂N*(CH₃)₃ groups as active sites.
The counter ion of the positively charged solid catalyst according to the invention can be selected from any negatively charged ion such as Cl⁻, Br⁻ or OH⁻. In all embodiments of the invention the counter ion is preferably OH⁻. The concentration of the active sites is preferably >0.7 eq/L, most preferably ≥ 0.8 eq/L. The solid catalysts for the processes according to the invention can be prepared by known methods to a person skilled in the art or are e.g. obtainable from Rohm & Haas under the tradename Amberlyst 26 OH or Ambersep 900.

Suitable ketones for the process according to the invention include acetone, methyl ethyl ketone (2-butanone), diethyl ketone (3-pentanone), methyl propyl ketone (i.e. 2-pentanone), 3-methylbutyraldehyde (i.e. isovaleraldehyd), methyl n-butyl ketone (2-hexanone), methyl isobutyl ketone, methyl-tert.-butyl ketone without being limited thereto. Preferably, the ketone is selected from acetone, 2-pentanone or 2-butanone.

Exemplary aldehydes for the process include paraformaldehyde, 3-methylbutyraldehyde, 3-methyl crotonic aldehyde, heptanal, citral, 3,7-dimethyl-2-octenal or 3,7-dimethyl-1-octanal without being limited thereto. Preferred aldehydes according to the invention are paraformaldehyd, heptanal, 3-methylbutyraldehyd and citral.

In a further preferred embodiment the invention relates to a process according to the invention wherein the aldehyde of formula (5) is citral.

Furthermore, the process according to the invention is particulary suitable for carrying out the aldol condensation of citral (formula (5) n = 1) with acetone (formula (4) R4 = methyl, R5 = hydrogen) to give ψ-ionone (formula (3) R4 = methyl, R5 = hydrogen, n = 1) respectively of citral (formula (5) n = 1) with 2-pentanone (formula (4) R4 = propyl R5 = hydrogen) to give 8,12-dimethyl-5,7,11-tridecatrien-4-one (formula (3) R4 = propyl, R5 = hydrogen, n = 1) as well as of 3-methylbutyraldehyde (formula (5) n = 0) with acetone to give as depicted in the scheme below;

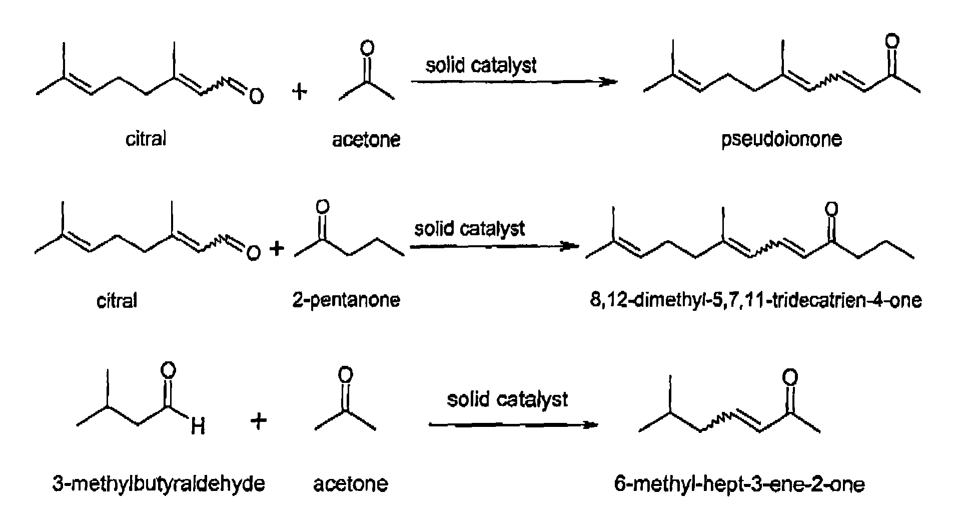

ψ-lonone, 8,12-dimethyl-5,7,11-tridecatrien-4-one as well as 6-methyl-hept-3-ene-2-one are important intermediates for vitamin and fragrance production e.g. for the production of timberone, geraniol, nerol, geranyl acetone, neryl acetone, linalool, dihydrolinalool, isonaline, vitamin E, vitamin A and β-carotene.

Thus, in a particular embodiment, the invention relates to a process according to the invention with all the preferences and definitions given herein wherein the alkylketone and the aldehyde are selected from
(i) citral and acetone and/ or
(ii) citral and 2-pentanone and/ or
(iii) citral and diethyl ketone and/ or
(iv) citral and 2-butanone and/ or
(v) 3-methylbutyraldehyde and acetone and/ or
(vi) 3-methylbutyraldehyde and 2-butanone and/ or
(vi) 3-methylbutyraldehyde and 2-pentanone and/ or
(vii) heptanal and 2-butanone and/ or
(viii) heptanal and acetone.

A particular advantage of the inventive process is the use of the solid catalyst which exhibits an exceptional stability and allows simple regeneration by filtration. Thus, the solid catalyst can be re-used while maintaining its activity. After filtration, the solid catalyst can be regenerated by methods known to a skilled person in the art. For example the solid catalyst can be regenerated by washing with a polar solvent such as methanol or ethanol followed by a treatment with aqueous NaOH (e.g. 1 M NaOH), After the basic treatment, the catalyst is washed with water until the eluate is neutral followed by washing with a polar solvent such as in particular ethanol. Afterwards the solid catalyst is ready for use in the process according to the invention.

The catalyst may be used as such or it may be suspended prior to its use. In a preferred embodiment of the invention the solid catalyst is suspended in a polar solvent such as methanol or ethanol, in particular ethanol. After termination of the reaction the catalyst can be recycled by simple technical measures such as filtration or decantation.

The amount of the solid catalyst used in the processes according to the invention is based on the amount of aldehyde. Usually the amount of the solid catalyst is selected in the in the range of 15 to 50 mol%, preferably from 20 to 40 mol%, in particular in the range of 25 to 35 mol%, most in particular 33 mol% based on the aldehyde, The mol% catalyst to be used is calculated on the basis of its active sites. Such amounts of the solid catalyst are sufficient to obtain high yields of desired product.

The process according to the invention can be carried out without an additional solvent or in the presence of an additional solvent. Suitable solvents for the aldol condensation reaction are polar protic solvents, e.g. methanol or ethanol. Preferably, the reaction is carried out using ethanol. In another preferred embodiment the reaction is carried out solvent-free.

The ratio of ketone to aldehyde is not critical for the reaction and can vary over a wide range, although the ketone is normally used as excess component in order to achieve high product selectivity in relation to the aldehyde. Good results are obtained if a molar ratio of the aldehyde to the ketone of 1:0.5 to 1: 50, preferably 1:1 to 1:30, most preferably in the range of 1:3 to 1:10, in particular in the range of 1:4 to 1:8, most in particular 1:8. Advantageously, an excess of the ketone is used in the process according to the invention.

The reaction can be performed at various temperatures and pressures the optimum being defined by the starting materials used. Generally, the reaction temperatures may vary from - 20 to 100°C. Preferably, the reaction temperature is selected in the range of 20 to 60°C, in particular in the range of 40 to 60°C, in particular 60°C. The pressures may vary from 0.1 to 100 bar absolute. Preferably, the reaction is carried out at atmospheric pressure.

The process according to the invention can in principle be carried out in any reactor suitable for heterogeneously catalyzed reactions. Without restricting generality, the following are mentioned by way of example: suspension reactor, stirred tank, stirred tank cascade, tubular reactor, shell-type reactor, shell and tube reactor, fixed-bed reactor, fluidized-bed reactor, reactive distillation column.

The invention is illustrated further by the examples without being limited thereto.

### Example 1

Paraformaldehyde (63.3 mmol, 1 equiv.) acetone (26 equiv.) and 25 wt.% (based on paraformaldehyde) Amberlyst A 26 OH are mixed in a round bottom flask and the flask is put into an oil bath, preheated to 50 °C. The reaction mixture is stirred for 23.5h, filtered and analyzed, resulting in 10% yield of hydroxybutan-2-one.

The same reaction is repeated using 25 wt.-% (based on paraformaldehyde) Ambersep 900 OH resulting in a yield of 4-hydroxybutane-2-one of 13%.

### Example 2

3 g of Amberlyst A 26 OH is stirred for 15 min in 50 ethanol. The solvent is decanted. After the addition of 50 ml ethanol the reaction mixture is heated to 40°C, respectively 60°C. Citral (0.65g, 11.1mmol) and a suitable amount of acetone in order to achieve the indicated molar ratios as shown in table 1 are added at once and the reaction mixture is stirred for 3 h at 40 °C/ 60°C. The reaction mixture is decanted and the ion exchanger washed twice with 25 ml ethanol. The combined organic phases are concentrated in vacuo at 40 °C and 30 mbar. The yield of pseudoionone is given in the table below. The catalyst is re-used for each run without further treatment.

**Table 1**

| **Run** | **Acetone citral 1:1*** | | **Acetone citral 4:1*** | | **Acetone citral 8:1*** | |
|---|---|---|---|---|---|---|
| | yield [%] 40°C | yield [%] 60°C | yield [%] 40°C | yield [%] 60°C | yield [%] 40°C | yield [%] 60°C |
| 1 | 23 | 50 | 54 | 62 | 66 | 68 |
| 2 | 18 | 38 | 53 | 60 | 65 | 67 |
| 3 | 13 | 32 | 46 | 60 | 62 | 69 |
| 4 | 6 | 17 | 32 | 62 | 49 | 77 |
| 5 | | | 10 | 39 | 36 | 43 |

| | | | | | | |
|---|---|---|---|---|---|---|
| * ratio based on the molar amount | | | | | | |

The reaction outlined above using citral and acetone has been repeated using a weak basic anion exchange, i.e. Amberlite IRA-96. However no conversion at all could be achieved.

### Example 3

Citral (6.00 g, 91.4%, 36.02 mmol) and acetone (9.28 g, 99.5 %, 158.98 mmol) are kept under argon in a 2-neck-flask. Ambersep 900 OH (1.08 g) is added and the reaction mixture is stirred at 62°C for 2.5 h. The reaction mixture is filtered and the ion exchanger washed once with acetone after the reaction. The combined organic phases are concentrated in vacuo at 40 °C and 30 mbar yielding 74% of pseudo-ionone. The reaction is repeated using the recycled catalyst from above without further treatment yielding in the second run 75% of pseudoionone.

### Example 4

Citral (2.00 g, 95.0%, 12.5 mmol) and 2-pentanone (4.80 g, 99.0%, 55.2 mmol) are stirred under argon in a 2-neck-flask at 60°C. After the addition of Ambersep 900 OH (360 mg) the mixture was stirred for 21 h at 60 °C. The reaction mixture is filtered, once washed with 2-pentanone and concentrated in vacuum yielding 95% of 8,12-dimethyl-trideca-5,7,11-trien-4-one.

¹H-NMR (300 MHz, CDCl₃): δ = 7.47 (dd, *J*=11.5 Hz, 14.2 Hz, HC(6)); 7.44 (dd, J= 11.5 Hz, 15.2 Hz, HC(6)): 6.11 (d, *J* =15.3 Hz, HC(5) or HC(7)); 6.07 (d, *J =* 15.2 Hz, HC(5) or HC(7)); 5.99 (d, *J =* 11.4 Hz, HC(5) or HC(7)); 5.12 (m, HC(11)); 2.53 (t, *J =* 7.3 Hz, H₂C(3) or H₂C(9)); 2.52 (t, *J* = 7.2 Hz, H₂C(3) or H₂C(9)); 2.32 (t, *J* = 7.9 Hz, H₂C(3) or H₂C(9)); 2.16 (s, CH₃), 1.90 (s, CH₃), 1.68 (m, CH₂, H₂C(2) and H₂C(10)); 1.61 (s, CH₃), 0.95 (t, *J*= 7.4 Hz, H₃C(1)); ¹³C-NMR (75 MHz, CDCl₃): 8 = 201.0 (CO), 150.9 (C=C), 150.9 (C=C), 138.6 (C=C), 138.4 (C=C), 132.6 (C=C), 132.2 (C=C), 127.6 (C=C), 127.4 (C=C), 124.7 (C=C), 123.8 (C=C), 123.3 (C=C), 123.2 (C=C), 42.8, 42.7, 40.4, 33.0, 26.9, 26.3, 25.7, 24.6, 18.0, 17.9, 17.7, 17.5, 13.9.

### Example 5

Isovaraldehyde (2.00 g, 98.0%, 22.8 mmol) and 2-pentanone (5.40 g, 99.5%, 92.5 mmol) are stirred under argon in a 2-neck-flask at 60°C. After the addition of Amberlyst A 26 (OH) (370 mg) the mixture was stirred for 200 min at 60 °C. The reaction mixture is filtered, once washed with acetone and concentrated in vacuum yielding 6-methyl-hept-3-ene2-one 18% and 4-hydroxy-6-methylheptan-2-one 64%.

## Claims

1. A process for the production of α,β-unsaturated ketones, the process comprising the step of reacting an aldhyde of formula (1) 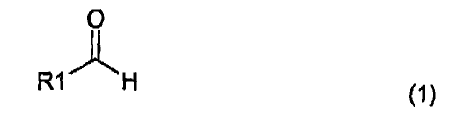 with a ketone of formula (2) 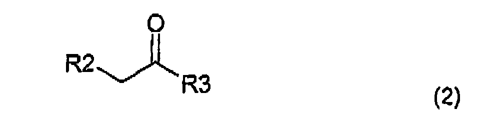 wherein R1 to R3 represent independently of each other hydrogen, a saturated or unsaturated, linear or branched hydrocarbons residue with 1 to 20 C-atoms or a cycloalkyl group with 4 to 12 C-atoms, which optionally may be substituted e.g. by one or several methoxy groups
in the presence of a strongly basic, anionic, macroreticular polymeric resin having
a)quaternary ammonium groups of the type -CH₂N⁺(CH₃)₃ as active sites, and
b) a surface area of 10-100 m²/g, preferably of 20 - 50 m²/g, and
c) an average pore diameter of 200-500 A.

2. The process according to claim 1 wherein R3 is methyl.

3. The process according to claim 1 or 2 for the production of α,β-unsaturated ketones of formula (3) 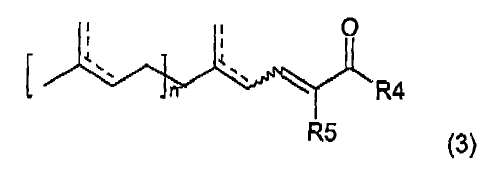 the process comprising reacting an alkylketone of formula (4) 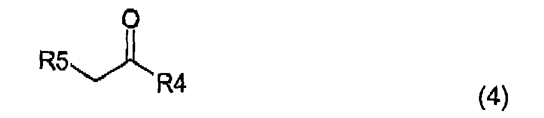 and an aldehyde of formula (5) 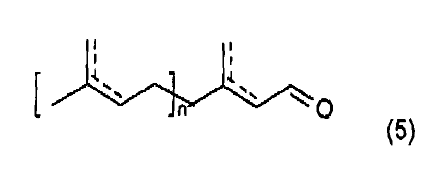 wherein
R4 represents a saturated or unsaturated, linear or branched hydrocarbons residue with 1 to 20 C-atoms and
R5 represents hydrogen, a saturated or unsaturated, linear or branched hydrocarbons residue with 1 to 20 C-atoms or a cycloalkyl group with 4 to 12 C-atoms, which optionally may be substituted e.g. by one or several methoxy or ether groups and
n represents a number from 0 to 4 and
the dotted lines indicate, independently of each other, either a saturated or a double bond.

4. The process according to claim 3 wherein R4 is a linear, saturated hydrocarbon residue with 1 to 5 C-atoms.

5. The process according to claim 3 or 4, wherein n represent a number from 1 to 2.

6. The process according to anyone of claim 3 to 5, wherein the aldehyde of formula (5) is citral.

7. The process according to claim 6 wherein the alkylketone is selected from acetone, 2-pentanone, diethyl ketone or 2-butanone

8. The process according to claim 1 wherein the aldehyde is 3-methylbutyraldehyd and the alkylketone is selected from acetone, 2-butanone or 2-pentanone.

9. The process according to any of claims 1 to 8, wherein the basic, anionic, macroreticular polymeric resin has a surface area of 30 m²/g and an average pore diameter of 290-300 A.

10. The process according to any of claims 1 to 9, wherein the strongly basic, anionic, macroreticular polymeric resin is a resin based on a crosslinked polystyrene divinylbenzene copolymer.

11. The process according to any of claim 1 to 10, wherein the molar ratio of the aldehyde to the ketone is in the range of 1:3 to 1:10, in particular in the range of 1:4 to 1:8, most in particular 1:8.

12. The process according to any of claims 1 to 11, wherein the strongly basic, anionic, macroreticular polymeric resin is filtrated and re-used in a subsequent reaction cycle.

13. The process according to any of claims 1 to 12, wherein the process is repeated 4 times using the recycled solid catalyst.
